# EUROPEAN PATENT APPLICATION

(11) **EP 4 717 277 A1**
(43) Date of publication of application: **01.04.2026**
(21) Application number: 24810327.7
(22) Date of filing: 17.05.2024
(51) Int. Cl.: A61K 47/68, A61K 47/65, A61K 31/4745, A61K 39/395, C07D 491/22, C07K 16/28, A61P 35/00, A61P 35/04

(54) **USE OF DRUG CONJUGATE IN TREATING TUMOR DISEASE AND METHOD THEREFOR**

(30) Priority: 22.05.2023 CN 202310579893
(71) Applicant: Sichuan Kelun-Biotech Biopharmaceutical Co., Ltd., Chengdu, Sichuan 611138 (CN)
(72) Inventor: GE, Junyou, Chengdu, Sichuan 611138 (CN); JIN, Xiaoping, Chengdu, Sichuan 611138 (CN); DIAO, Yina, Chengdu, Sichuan 611138 (CN); YANG, Yalan, Chengdu, Sichuan 611138 (CN); LIU, Gesha, Chengdu, Sichuan 611138 (CN); FU, Yujie, Chengdu, Sichuan 611138 (CN); JING, Dian, Chengdu, Sichuan 611138 (CN); ZHONG, Hongbo, Chengdu, Sichuan 611138 (CN)
(74) Representative: Ipsilon
(86) International application number: PCT/CN2024/093937
(87) International publication number: WO 2024/240084

(57) **Abstract**

The present invention relates to a use of drug conjugates for treating tumor diseases and method thereof. Specifically, the present invention provides the use of the bioactive molecule conjugate represented by Formula (I) in treating tumor diseases and method thereof, wherein the tumor diseases are particularly preferably unresectable locally advanced or metastatic solid tumors that are refractory to current standard treatment, especially non-small cell lung cancer.

## Description

The present application is based on and claims priority to CN Application No. 202310579893.9 filed on May 22, 2023, the disclosure of which is incorporated herein by reference in its entirety.

### TECHNICAL FIELD

The present application relates to the use of a drug conjugate in treatment of tumor diseases and method thereof, especially in treatment of unresectable locally advanced or metastatic solid tumors that are refractory according to existing treatment standards, such as tumors that have failed and/or recurred after first-line chemotherapeutics, tumors that have failed and/or recurred after radiotherapy, and/or tumors that have failed and/or recurred after targeted therapeutics, specifically non-small cell lung cancer.

### BACKGROUND

Cancer is a big burden of global public health. In the United States, cancer is still the second leading cause of death after cardiovascular diseases. In China, the number of cancer cases is also on the rise. In 2019, the number of new cases of malignant tumors in China was about 4.4 million, and the death toll was about 2.624 million. The rising number of cancer patients and deaths will lead to the expansion of the overall size of the tumor treatment market.

Chemotherapy is one of the main means of cancer treatment, but traditional chemotherapeutics are not specific in recognizing tumors, which is easy to injure normal cells and cause serious adverse reactions in patients. In order to improve the survival rate of cancer patients, it is urgent to innovate the treatment methods to match the progress of detection and diagnosis. Although great progress has been made in many indications, the mortality resulting from some of the most refractory solid tumors has not been improved significantly since the 1970s, and more effective treatments with fewer side effects are still needed. Molecular targeted drugs are an important direction of drug design at present.

Monoclonal antibody drugs have the advantages of strong targeting, high specificity and low incidence of serious adverse reactions, but their molecular weight is high and their therapeutic effect is limited when used alone. ADCs (antibody-drug conjugates) are drugs that couple monoclonal antibodies with different numbers of small molecular cytotoxins (effector molecules) through chemical linkers. After entering the body, ADC molecules can bind to antigens on the surface of target cells through the guidance of monoclonal antibodies, and enter the target cells.

The ADC molecules entering the cells can release effector molecules through chemical and/or enzymatic actions to achieve the goal of destroying the target cells. ADC drugs combine the advantages of strong targeting of monoclonal antibodies and high activity of small molecular toxins, which can not only reduce the toxic and side effects of small molecular cytotoxins, but also improve the efficacy of drugs.

At present, a variety of ADC drugs are commercially available worldwide, and their indications cover leukemia, lymphoma and breast cancer, etc. However, there are still some problems in the pharmacokinetics and safety of some ADC drugs, which may lead to serious adverse reactions in patients after use. Also, their response rate for some metastatic, recurrent and/or refractory cancers still needs to be further improved. Therefore, there is still a need to develop use or treatment methods for ADC drugs to treat these metastatic, recurrent, and/or refractory cancers, so as to maximize their efficacy and minimize their toxicity to meet the drug demand of cancer patients.

### SUMMARY

The present invention provides a use of a bioactive molecule conjugate represented by Formula (I) in the manufacture of a medicament for treating a subject with a tumor disease, especially a subject with a recurrent or refractory locally advanced or metastatic non-small cell lung cancer;

{D-[L₁-(L₂)ₘ₁-(L₃)ₘ₂-(L₄)ₘ₃-E]}_{γ}-A Formula (I)

wherein,
L₁ is 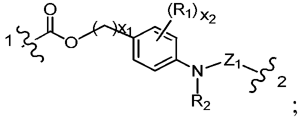 wherein R₁ and R₂ are each independently hydrogen (such as protium or deuterium), halogen, carboxylate, sulfonate, cyano, C₁₋₆ alkyl, halogenated C₁₋₆ alkyl, cyano-substituted C₁₋₆ alkyl (such as-CH₂CN), C₁₋₆ alkoxy, C₂₋₁₀ alkenyl or C₂₋₁₀ alkynyl; Z₁ is an amino acid or a peptide consisting of 2-10 amino acids; x₁ and x₂ are each independently 0, 1, 2, 3, 4, 5 or 6; and the position 1 of L₁ is attached to D, and the position 2 of L₁ is attached to L₂;
L₂ is 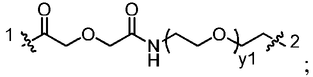 wherein y1 is 0, 1, 2, 3, 4, 5, 6, 7, 8, 9 or 10; and the position 1 of L₂ is attached to L₁, and the position 2 of L₂ is attached to L₃;
L₃ is 5-12-membered heteroaromatic ring;
L₄ is 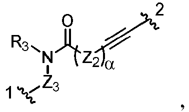 wherein Z₂ is selected from C₁₋₆ alkylene, C₂₋₁₀ alkenylene, C₂₋₁₀
alkynylene, and C₃₋₈ cycloalkylene; R₃ is selected from H and C₁₋₆ alkyl; Z₃ is absent or is C₁₋₆ alkylene; or, R₃ and Z₃ together with the nitrogen atom to which they are attached form 4-8membered heterocyclyl; α is 0, 1, 2, 3, 4, 5 or 6, and the position 2 of L₄ is attached to E, and the position 1 of L₄ is attached to L₃;
E is 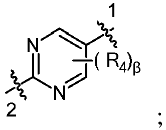 wherein each R₄ is independently hydrogen (such as protium or deuterium), β is 0, 1 or 2, and the position 2 of E is attached to A (such as with sulfhydryl on A), and the position 1 of E is attached to L₄;
m₁, m₂ and m₃ are each independently 0, 1, 2, 3, 4, 5, 6, 7, 8, 9 or 10;
D is a bioactive molecular fragment;
γ refers to the number of moiety {D-[L₁-(L₂)ₘ₁-(L₃)ₘ₂-(L₄)ₘ₃-E}attached to A through a sulfide bond, and is selected from integers from 1 to 10; preferably, γ is selected from integers from 3 to 8 (such as 3, 4, 5, 6, 7 or 8); and
A is a monoclonal antibody against Trop-2 or an antigen-binding fragment thereof.

In some embodiments, the tumor disease is an unresectable locally advanced or metastatic solid tumor that has failed standard treatments, has no standard treatment regimen, or is not suitable for standard treatments at the present stage. In some embodiments, the standard treatment refers to the standard treatment regimens for the tumor disease as recommended by NCCN guidelines and CSCO guidelines for diagnosis and treatment.

In some embodiment, the tumor disease is a tumor that has failed and/or recurred after first line chemotherapeutics. In some embodiments, the first-line chemotherapeutics refer to the first line chemotherapeutics for the tumor disease as recommended by NCCN guidelines and CSCO guidelines for diagnosis and treatment.

In some embodiment, the tumor disease is a tumor that has failed and/or recurred after radiotherapy. In some embodiments, the radiotherapy refers to the radiotherapy regimens for the tumor disease as recommended by NCCN guidelines and CSCO guidelines for diagnosis and treatment.

In some embodiment, that tumor disease is a tumor that has failed and/or recurred after targeted drugs or immunotherapy. In some embodiments, the targeted drug or immunotherapy refers to the targeted drug or immunotherapy for the tumor disease as recommended by NCCN guidelines and CSCO guidelines for diagnosis and treatment.

In some embodiments, the tumor disease includes but is not limited to breast cancer, gastric cancer, lung cancer, ovarian cancer, urinary tract epithelial cancer, esophageal cancer, liver cancer, colorectal cancer, cervical cancer, endometrial cancer, pancreatic cancer, bladder cancer, or brain tumor; preferably breast cancer (such as triple negative breast cancer or Her2 positive breast cancer), ovarian cancer (such as ovarian epithelial cancer), gastric cancer, lung cancer, pancreatic cancer, bladder cancer, or urinary tract epithelial cancer; more preferably, the tumor disease is triple negative breast cancer, Her2 positive breast cancer, ovarian cancer, gastric cancer, lung cancer or pancreatic cancer; further preferably, the tumor disease is triple negative breast cancer, Her2 positive breast cancer, ovarian cancer or gastric cancer.

In some embodiments, the tumor disease is breast cancer.

In some embodiments, the breast cancer includes but is not limited to the following types: Luminal A, Luminal B, Her2-positive, triple-negative, or HR+/Her2- breast cancer.

In some embodiments, the breast cancer includes but is not limited to the following types: Luminal A, Luminal B, Her2-positive, and triple-negative breast cancer.

In some embodiments, the tumor disease is triple-negative breast cancer.

In some embodiments, the tumor disease is Her2-positive breast cancer.

In some embodiments, the tumor disease is HR+/Her2- breast cancer.

In some embodiments, the tumor disease is ovarian cancer.

In some embodiments, the ovarian cancer includes but is not limited to the following types: platinum sensitive type and platinum resistant type.

In some embodiments, the tumor disease is gastric cancer.

In some embodiments, the gastric cancer includes but is not limited to the following types: adenocarcinoma, adenosquamous carcinoma, squamous cell carcinoma, undifferentiated carcinoma and neuroendocrine tumor.

In some embodiments, the tumor disease is pancreatic cancer.

In some embodiments, the pancreatic cancer includes but is not limited to the following types: epithelial tumor, exocrine tumor, borderline tumor, ductal adenocarcinoma, endocrine tumor, mature teratoma, mesenchymal tumor, malignant lymphoma and secondary tumor.

In some embodiments, the tumor disease is bladder cancer.

In some embodiments, the bladder cancer includes but is not limited to the following types: urothelial (transitional cell) carcinoma, squamous cell cancer and adenocarcinoma.

In some embodiments, the tumor disease is urinary tract epithelial cancer.

In some embodiments, the urinary tract epithelial cancer includes but is not limited to the following types: basal type, lumen type and wild type urinary tract epithelial cancers.

In some embodiments, the tumor disease is lung cancer.

In some embodiments, the lung cancer includes but is not limited to the following types: small cell lung cancer (SCLC) and non-small cell lung cancer (NSCLC).

In some embodiments, the lung cancer is recurrent or refractory locally advanced or metastatic non-small cell lung cancer.

In some embodiments, the lung cancer is EGFR wild-type non-small cell lung cancer.

In some embodiments, the subject with EGFR wild-type non-small cell lung cancer has previously received a median of two lines of therapy.

In some embodiments, the subject with EGFR wild-type non-small cell lung cancer has previously received a platinum-based chemotherapy combined with anti-PD-1/L1 monoclonal antibody therapy.

In some embodiments, the lung cancer is EGFR-mutated non-small cell lung cancer.

In some embodiments, the EGFR-mutated NSCLC includes exon 19 deletions or exon 21 L858R mutations.

In some embodiments, the subject with EGFR-mutated NSCLC has previously received treatment with a first-generation or second-generation epidermal growth factor receptor-tyrosine kinase inhibitor (EGFR-TKI), and confirmed by tumor biopsy that exon 20 T790M mutation is negative after treatment failure, or the subject with EGFR-mutated NSCLC has previously received treatment with a third-generation EGFR-TKI (irrespective of T790M mutation status).

In some embodiments, the EGFR-mutated non-small cell lung cancer is resistant to EGFR-TKI treatment.

In some embodiments, the conjugate has the following structure:
L₁ is selected from 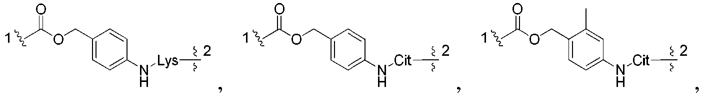 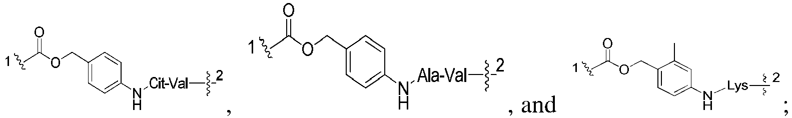 and the position 1 of L₁ is attached to D, and the position 2 of L₁ is attached to L₂;
L₂ is 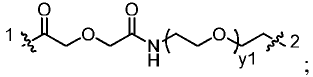 wherein y₁ is 3, 4, 5, 6, 7, 8, 9 or 10; and the position 1 of L₂ is attached to L₁, and the position 2 of L₂ is attached to L₃;
L₃ is selected from 5-6-membered heteroaromatic rings, such as pyrazole or triazole;
L₄ is 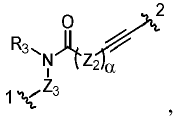 wherein Z₂ is selected from C₁₋₃ alkylene; R₃ is H; Z₃ is selected from C₁₋₃ alkylene; α is 0, and the position 2 of L₄ is attached to E, and the position 1 of L₄ is attached to L₃;
E is 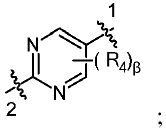 wherein each R₄ is independently hydrogen (such as protium or deuterium), β is 0, 1 or 2, and the position 2 of E is attached to A (such as with sulfhydryl on A), and the position 1 of E is attached to L₄; m₁, m₂ and m₃ are all 1;
the bioactive molecule is selected from 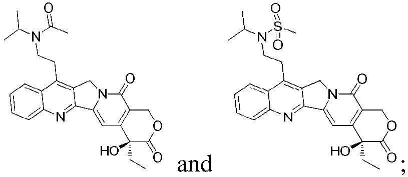 preferably, the bioactive molecule is attached to the position 1 of L₁ through its own hydroxyl; γ is selected from integers from 3 to 8 (for example, 3, 4, 5, 6, 7 or 8);
A is Sacituzumab or an antigen-binding fragment thereof.

In some embodiments, the conjugate has the following structure:
L₁ is selected from 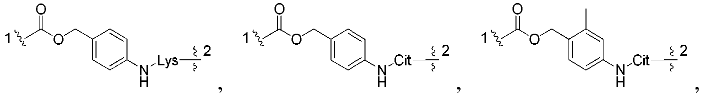 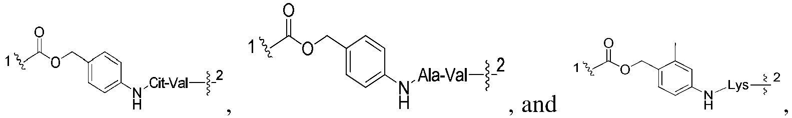 and the position 1 of L₁ is attached to D, and the position 2 of L₁ is attached to L₂;
L₂ is 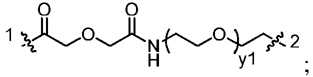 wherein y₁ is 3, 4, 5, 6, 7, 8, 9 or 10; and the position 1 of L₂ is attached to L₁, and the position 2 of L₂ is attached to L₃;
L₃ is selected from 5-6-membered heteroaromatic rings, such as pyrazole or triazole;
L₄ is 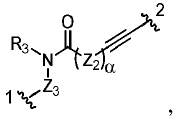 wherein Z₂ is selected from C₁₋₃ alkylene; R₃ is H; Z₃ is selected from C₁₋₃ alkylene; α is 1, and the position 2 of L₄ is attached to E, and the position 1 of L₄ is attached to L₃;
E is 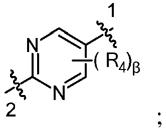 wherein each R₄ is independently hydrogen (such as protium or deuterium), β is 0, 1 or 2, and the position 2 of E is attached to A (such as with sulfhydryl on A), and the position 1 of E is attached to L₄; m₁, m₂ and m₃ are all 1;
the bioactive molecule is selected from 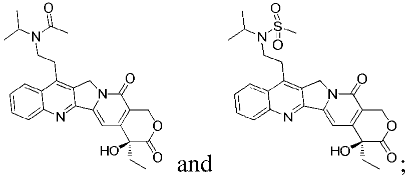 preferably, the bioactive molecule is attached to the position 1 of L₁ through its own hydroxyl; γ is selected from integers from 3 to 8 (for example, 3, 4, 5, 6, 7 or 8); and A is Sacituzumab or an antigen-binding fragment thereof.

In some embodiments, D is selected from

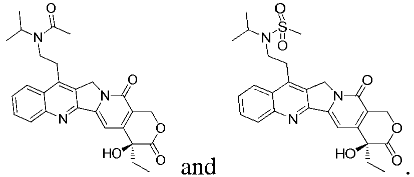

In some technical solutions, the conjugate is conjugate A with a structure represented by the following formula: 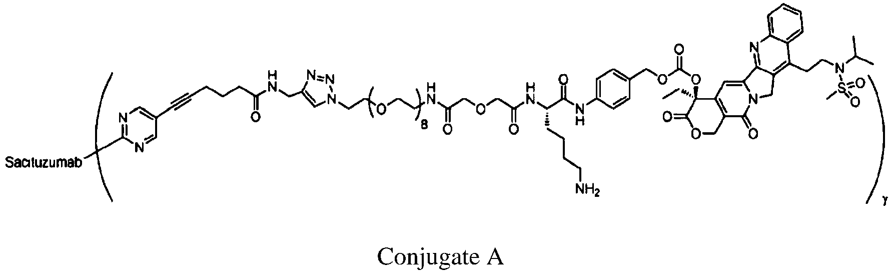 wherein γ is an integer from 1 to 10; preferably, γ is selected from integers from 5 to 8.

The present invention provides a use of a composition comprising the bioactive molecule conjugate of Formula (I) as described herein. The composition of the bioactive molecule conjugate as described herein has a DAR of about 1 to about 10, or any subrange therebetween, for example: about 1 to 2, about 1 to 3, about 1 to 4, about 1 to 5, about 1 to 6, about 1 to 7, about 1 to 8, about 1 to 9, about 1 to 10, about 2 to 3, about 2 to 4, about 2 to 5, about 2 to 6, about 2 to 7, about 2 to 8, about 2 to 9, about 2 to 10, about 3 to 4, about 3 to 5, about 3 to 6, about 3 to 7, about 3 to 8, about 3 to 9, about 3 to 10, about 4 to 5, about 4 to 6, about 4 to 7, about 4 to 8, about 4 to 9, about 4 to 10, about 5 to 6, about 5 to 7, about 5 to 8, About 5 to 9, about 5 to 10, about 6 to 7, about 6 to 8, about 6 to 9, about 6 to 10, about 7 to 8, about 7 to 9, about 7 to 10, about 8 to 9, about 8 to 10, or about 9 to 10.

In certain embodiments, the composition of the bioactive molecule conjugate as described herein has a DAR of about 3 to 9, for example, about 3.0 to 3.5, about 3.0 to 4.0, about 3.0 to 4.5, about 3.0 to 5.0, about 3.0 to 6.0, about 3.5 to 4.0, about 3.5 to 4.5, about 3.5 to 5.0, about 3.5 to 5.5, about 3.5 to 6.0, about 3.5 to 6.5, about 4.0 to 4.5, about 4.0 to 5.0, about 4.0 to 5.5, about 4.0 to 6.0, about 4.0 to 6.5, about 4.0 to 7.0, about 4.0 to 8.0, about 4.5 to 5.0, about 4.5 to 5.5, about 4.5 to 6.0, about 4.5 to 6.5, about 4.5 to 7.0, about 4.5 to 7.5 , about 5.0 to 8.0, about 5.5 to 6.0, about 5.5 to 6.5, about 5.5 to 7.0, about 5.5 to 7.5, about 5.5 to 8.0, about 6.0 to 6.5, about 6.0 to 7.0, about 6.0 to 7.5, about 6.0 to 8.5, about 6.5 to 7.0, about 6.5 to 7.5, about 6.5 to 8.5, about 7.0 to 7.5.

In some embodiments, the composition of the bioactive molecule conjugate as described herein has a DAR value of about 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0.

In another aspect, the present invention provides a method for treating a tumor disease, in particular a recurrent or refractory non-small cell lung cancer, wherein the method comprises a step of administering to a subject in need thereof a therapeutically effective amount of the bioactive molecule conjugate of Formula (I) as described above and/or a pharmaceutical composition comprising the bioactive molecule conjugate of Formula (I).

In some embodiments, the tumor disease is an unresectable locally advanced or metastatic solid tumor that has failed standard treatments, has no standard treatment regimen, or is not suitable for standard treatments at the present stage. In some embodiments, the standard treatment refers to the standard treatment regimens for the tumor disease as recommended by NCCN guidelines and CSCO guidelines for diagnosis and treatment.

In some embodiment, that tumor disease is a tumor that has failed and/or recurred after first line chemotherapeutics. In some embodiments, the first-line chemotherapeutics refer to the first-line chemotherapeutics for the tumor disease as recommended by NCCN guidelines and CSCO guidelines for diagnosis and treatment.

In some embodiment, that tumor disease is a tumor that has failed and/or recurred after targeted drugs or immunotherapy. In some embodiments, the targeted drug or immunotherapy refers to the targeted drug or immunotherapy for the tumor disease as recommended by NCCN guidelines and CSCO guidelines for diagnosis and treatment.

In some embodiments, the tumor disease includes but is not limited to breast cancer, gastric cancer, lung cancer, ovarian cancer, urinary tract epithelial cancer, esophageal cancer, liver cancer, colorectal cancer, cervical cancer, endometrial cancer, pancreatic cancer, bladder cancer, or brain tumor; preferably breast cancer (such as triple negative breast cancer or Her2 positive breast cancer), ovarian cancer (such as ovarian epithelial cancer), gastric cancer, lung cancer, pancreatic cancer, bladder cancer, or urinary tract epithelial cancer; more preferably, the tumor disease is triple negative breast cancer, Her2 positive breast cancer, ovarian cancer, gastric cancer, lung cancer or pancreatic cancer; further preferably, the tumor disease is triple negative breast cancer, Her2 positive breast cancer, ovarian cancer or gastric cancer.

In some embodiments, the tumor disease is breast cancer.

In some embodiments, the breast cancer includes but is not limited to the following types: Luminal type A, Luminal type B, Her-2 positive type, and triple negative type.

In some embodiments, the tumor disease is triple negative breast cancer.

In some embodiments, the tumor disease is Her2 positive breast cancer.

In some embodiments, the tumor disease is HR-positive and Her2-negative breast cancer (HR+/Her2- breast cancer).

In some embodiments, the tumor disease is ovarian cancer.

In some embodiments, the ovarian cancer includes but is not limited to the following types: platinum sensitive type and platinum resistant type.

In some embodiments, the tumor disease is gastric cancer.

In some embodiments, the gastric cancer includes but is not limited to the following types: adenocarcinoma, adenosquamous carcinoma, squamous cell carcinoma, undifferentiated carcinoma and neuroendocrine tumor.

In some embodiments, the tumor disease is pancreatic cancer.

In some embodiments, the pancreatic cancer includes but is not limited to the following types: epithelial tumor, exocrine tumor, borderline tumor, ductal adenocarcinoma, endocrine tumor, mature teratoma, mesenchymal tumor, malignant lymphoma and secondary tumor.

In some embodiments, the tumor disease is bladder cancer.

In some embodiments, the bladder cancer includes but is not limited to the following types: urothelial (transitional cell) carcinoma, squamous cell cancer and adenocarcinoma.

In some embodiments, the tumor disease is urinary tract epithelial cancer.

In some embodiments, the urinary tract epithelial cancer includes but is not limited to the following types: basal type, lumen type and wild type urinary tract epithelial cancers.

In some embodiments, the tumor disease is lung cancer.

In some embodiments, the lung cancer includes but is not limited to the following types: small cell lung cancer, non-small cell lung cancer.

In some embodiments, the lung cancer is recurrent or refractory locally advanced or metastatic non-small cell lung cancer.

In some embodiments, the lung cancer is EGFR wild-type non-small cell lung cancer.

In some embodiments, the subject with EGFR wild-type non-small cell lung cancer has previously received a median of two lines of therapy.

In some embodiments, the subject with EGFR wild-type non-small cell lung cancer has previously received platinum-based chemotherapy combined with anti-PD-1/L1 monoclonal antibody therapy.

In some embodiments, the lung cancer is EGFR-mutated non-small cell lung cancer.

In some embodiments, the EGFR-mutated NSCLC includes exon 19 deletions or exon 21 L858R mutations.

In some embodiments, the subject with EGFR-mutated NSCLC has previously received treatment with a first-generation or second-generation epidermal growth factor receptor-tyrosine kinase inhibitor (EGFR-TKI), and confirmed by tumor biopsy that the exon 20 T790M mutation is negative after treatment failure, or the subject with EGFR-mutated NSCLC has previously received treatment with a third-generation EGFR-TKI (irrespective of T790M mutation status).

In some embodiments, the EGFR-mutated non-small cell lung cancer is resistant to EGFR-TKI treatment.

In some embodiments, the subject is a subject with recurrent or refractory locally advanced or metastatic non-small cell lung cancer and other tumor.

In some embodiments, the subject is a subject with EGFR wild-type non-small cell lung cancer, or a subject with EGFR-mutated non-small cell lung cancer and is resistant to EGFR-TKI treatment.

In some embodiments, the method for treating tumor disease further comprises evaluating the tumor per RECIST 1.1.

In some embodiments, the evaluation cycle is 6 to 8 weeks, for example, 8 weeks.

In some embodiments, the pharmaceutical composition comprises the bioactive molecule conjugate and a pharmaceutically acceptable carrier and/or excipient.

In some embodiments, the bioactive molecule conjugate or the pharmaceutical composition is administered once every 7 to 35 days, preferably once every 7 to 28 days, for example, once every 7 days, 14 days, 21 days, 28 days, or 35 days.

In some embodiments, the bioactive molecule conjugate or the pharmaceutical composition is administered once every 14 days.

In some embodiments, the conjugate or the pharmaceutical composition is administered by the following routes: oral administration, transdermal injection, rectal administration, transmucosal administration, intramuscular injection, intramedullary injection, intravenous injection, or intraperitoneal injection, preferably intravenous injection.

In some embodiments, the dose of the bioactive molecule conjugate for each administration based on the subject's body weight is 1 mg/kg to 30 mg/kg; preferably 1 mg/kg to 20 mg/kg; more preferably 2 mg/kg to 12 mg/kg; further preferably 2-5 mg/kg, 4-7 mg/kg, 6-9 mg/kg, 8-11 mg/kg, 10-13 mg/kg, or 12-15 mg/kg; for example: 2 mg/kg, 2.5 mg/kg, 3 mg/kg, 3.5 mg/kg, 4 mg/kg, 4.5 mg/kg, 5 mg/kg, 5.5 mg/kg, 6 mg/kg, 6.5 mg/kg, 7 mg/kg, 7.5 mg/ kg, 8 mg/kg, 8.5 mg/kg, 9 mg/kg, 9.5 mg/kg, 10 mg/kg, 11 mg/kg or 12 mg/kg.

In some embodiments, the dose of the bioactive molecule conjugate for each administration based on the subject's body weight is 4 mg/kg or 5 mg/kg.

In some embodiments, the dose of the bioactive molecule conjugate or its composition for each administration based on the subject's body weight is 1 mg/kg to 20 mg/kg, and is administered once every 7 to 28 days.

In some embodiments, the lung cancer is EGFR wild-type non-small cell lung cancer, or EGFR-mutated non-small cell lung cancer and is resistant to EGFR-TKI treatment.

In some embodiments, the lung cancer is EGFR wild-type non-small cell lung cancer, or EGFR-mutated non-small cell lung cancer and is resistant to EGFR-TKI treatment, and the dose of the bioactive molecule conjugate or its composition for each administration based on the subject's body weight is 5 mg/kg, and is administered once every 2 weeks.

In some embodiments, the administration regimen of the bioactive molecule conjugate is divided into one or more administration stages (e.g., one stage, two stages, three stages, or four stages), and the administration cycle and administration dose of each stage are independently selected from the administration cycle or dose described above. In some embodiments, the use or method of the present invention causes tumor elimination or volume reduction.

In some embodiments, the use or method of the present invention causes a reduction in tumor volume by at least 5%, at least 10%, at least 15%, at least 20%, at least 30% or at least 40%.

On the other hand, the present invention provides the bioactive molecule conjugate represented by Formula (I) or composition thereof for use in treating a tumor disease, wherein the bioactive molecule conjugate or the composition and the tumor disease are as described above.

### Definitions

Unless otherwise defined below, all technical and scientific terms used herein are intended to have the same meaning as commonly understood by those skilled in the art. References to technologies used herein are intended to refer to technologies commonly understood in the art, including those changes to technologies obvious to those skilled in the art or replacements of equivalent technologies. Although it is believed that the following terms are well understood by those skilled in the art, the following definitions are set forth to better explain the present invention.

Drug-antibody ratio (DAR) refers to the average loading of small molecular toxin drugs by antibodies in conjugates. Although for a specific conjugate molecule, the binding ratio of the small molecule toxin drug moiety to the antibody moiety has an exact value, it should be understood that when used to describe a sample containing many molecules, this value refers to an average value calculated according to the percentages of different specific conjugate molecules, and this average loading is called the average coupling ratio or "DAR" herein.

The term "subject" refers to a mammal, such as a primate mammal, such as a human. In some embodiments, the subject (e.g., human) has a tumor or cancer described herein, or is at risk of having the tumor or cancer.

NCCN guidelines refer to the clinical practice guidelines for various malignant tumors issued by the National Comprehensive Cancer Network.

CSCO guidelines for diagnosis and treatment refer to the guidelines for clinical diagnosis and treatment of various malignant tumors issued by the Chinese Society of Clinical Oncology (CSCO).

The term "platinum-based chemotherapy" refers to a chemotherapy using a platinum drug, where the platinum drug is selected from Cisplatin, Carboplatin, Sulfatodiaminocyclohexane platinum, Nedaplatin, Oxaliplatin, Lobaplatin, Satraplatin, Miboplatin, Enloplatin, Iproplatin or Dicycloplatin.

"First-generation EGFR-TKI inhibitor" is, for example, Erlotinib or Gefitinib. "Second-generation EGFR-TKI inhibitor" is, for example, Afatinib or Vizimpro (Dacomitinib). "Second-generation EGFR-TKI inhibitor" is, for example, Osimertinib, Rociletinib, Olmutinib or AC0010.

Objective Response Rate (ORR) refers to the proportion of patients whose tumors have shrunk to a certain extent and remain at this extent for a certain period of time, including CR and PR cases.

The objective response of tumors is evaluated by Response Evaluation Criteria in Solid Tumors Version 1.1 (RECIST 1.1). Subjects must be accompanied with measurable tumor lesions at baseline. According to the criteria of RECIST 1.1, the evaluation criteria of efficacy are divided into complete response (CR), partial response (PR), stable disease (SD) and progressive disease (PD).

Progressive Disease (PD): with the minimum value in the sums of the diameters of all the measured target lesions as reference, the sum of the diameters is relatively increased by at least 20% (if the baseline measurement value is the minimum, the baseline value will be taken as reference); in addition, the absolute value of the sum of diameters must be increased by at least 5 mm (the appearance of one or more new lesions is also regarded as progressive disease).

Stable Disease (SD): The reduction of the target lesion does not reach PR, and its increase does not reach PD level, the state falls in between, and the minimum sum of diameters could be used as a reference in the study.

Partial Response (PR): The sum of target lesions decreases by at least 30% compared with the baseline.

Complete Response (CR): All target lesions disappear, and the short diameter of all pathological lymph nodes (including target nodules and non-target nodules) must be reduced to <10 mm.

Disease Control Rate (DCR): The percentage of evaluable cases with pathologically confirmed disease that demonstrate disease relief (including complete response and partial response, PR + CR) or stable disease (SD) after treatment, with such responses sustained for at least four 4 weeks, per RECIST criteria.

Duration of Response (DoR): It refers to the time from the first assessment of CR or PR to the first assessment of PD or death from any cause.

Progression-Free Survival (PFS): The time from randomization or first dose of the study drug to the first evidence of disease progression or death from any cause.

Dose Limiting Toxicities (DLTs): the toxic and side effects of a drug that become the main reason to limit the continuous increase of drug dose.

Adverse Event (AE): refers to any adverse medical event that occurs after a patient or a clinical research subject receives a drug, but it is not necessarily causally related to the treatment.

Treatment Emergent Adverse Event (TEAE): refers to any AE that occurs or worsens at or after the first dosing.

### BRIEF DESCRIPTION OF THE DRAWINGS

The drawing described herein is used to provide a further understanding of the present invention and constitute a part of the present application. The illustrative examples of the present invention and their descriptions are used to explain the present invention and do not constitute an undue limitation of the present invention. In the drawings:

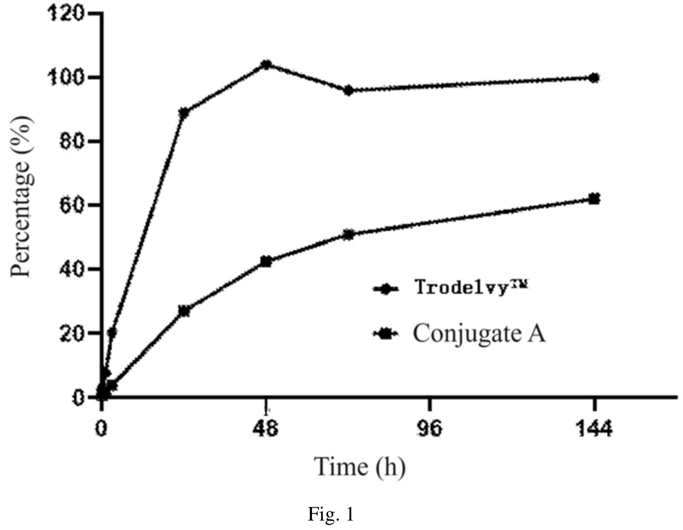

Fig. 1 shows the in vitro plasma stability of the conjugate A and a commercially available drug Trodelvy^{™}.

### DETAILED DESCRIPTION

In the following, the technical solutions in the examples of the present invention will be clearly and completely described with reference to the drawings in the examples of the present invention. Obviously, the described examples are only a part of but not all the examples of the present invention. The following description of at least one exemplary example is merely illustrative in nature and is in no way intended to limit the present invention, its applications or uses. Based on the examples in the present invention, all other examples obtained by those of ordinary skill in the art without creative work fall within the scope of protection of the present invention.

Example 1. 4-((S)-2-(4-aminobutyl)-35-(4-((6-(2-(methylsulfonyl)pyrimidin-5-yl)hexan-5-amido)methyl)-1H-1,2,3-triazol-1-yl)-4,8-dioxo-6,12,15,18,21,24,27,30,33-nonaoxa-3,9-diazapentatriacontanamino)benzyl((S)-4-ethyl-11-(2-(N-isopropylmethylsulfonamido)ethyl)3,14-dioxo-3,4,12,14-tetrahydro-1H-pyrano[3',4':6,7]indolizino[1,2-b]quinolin-4-yl)carbonate

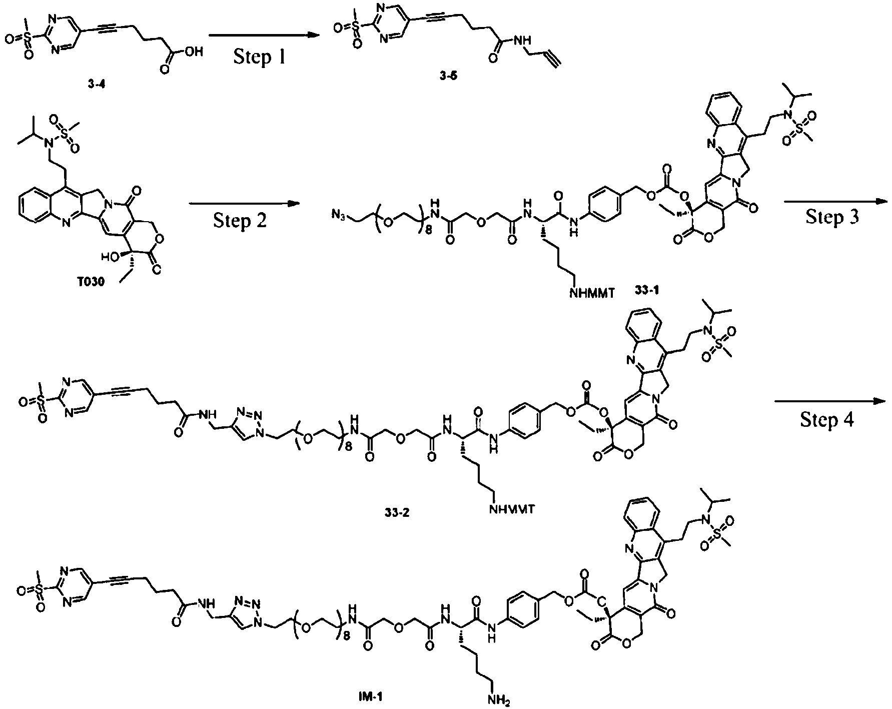

### Step 1: Synthesis of 6-(2-(methylsulfonyl)pyrimidin-5-yl)-N-(propan-2-alkyn-1-yl)hexa-5-alkynamide

At 25°C, prop-2-yn-1-amine (189 mg, 3.4 mmol) and compound 3-4 (800 mg, 2.83 mmol) were dissolved in dichloromethane (10 mL), N,N-diisopropylethylamine (738 mg, 5.67 mmol) and O-(7-azabenzotriazol-1-yl)-N,N,N',N'-tetramethylurea hexafluorophosphate (1.63 g, 4.25 mmol) were added in sequence, and the mixture was stirred to react for 2 h. The reaction mixture was concentrated under reduced pressure, and the residue was purified by rapid silica gel column (ethyl acetate/petroleum ether=3/1) to obtain the title compound (700 mg). ESI-MS (m/z): 306.1 [M+H]+.

### Step 2: Synthesis of 4-((S)-35-azido-2-(4-((4-methoxyphenyl)diphenylmethyl)amino)butyl)4,8-dioxo-6,12,15,18,21,24,27,30,33-nonaoxa-3,9-diazapentatriacontanamino)benzyl((S)-4-ethyl-11-(2-(N-isopropylmethanesulfonamido)ethyl)-3,14-dioxo-3,4,12,14-tetrahydro-2H-pyrano[2,3-b]-1H-pyrano[3',4':6,7]indazino[1,2-b]quinolin-4-yl)carbonate

Under the protection of nitrogen at 25°C, T-030 (250 mg, 0.49 mmol) was dissolved in dichloromethane (10 mL), and cooled to 0°C, then 4-dimethylaminopyridine (478 mg, 3.91 mmol) in dichloromethane (3 mL) was added, and then triphosgene (72 mg, 0.24 mmol) in dichloromethane (10 mL) was slowly added dropwise. Thereafter, the mixture was stirred at 0°C for 20 min and reaction mixture was blown with nitrogen for 20 min. (S)-2-(32-azido-5-oxo-3,9,12,15,18,21,24,27,30-nonaoxa-6-azatriacetamidyl)-N-(4-(hydroxymethyl)phenyl)-6-(((4-methoxyphenyl)diphenylmethyl)amino)acetamide (518 mg, 0.49 mmol) in dichloromethane (7 mL) was added. Thereafter, the mixture was stirred to react for 1 h. The reaction mixture was concentrated under reduced pressure, and the residue was purified by preparative HPLC to obtain the title compound (500 mg). ESI-MS (m/z): 1597.5[M+H]+.

### Step 3: Synthesis of (S)-4-ethyl-11-(2-(N-isopropylsulfonamido)ethyl)-3,14-dioxo-3,4,12,14-tetrahydro-1H-pyrano[3',4':6,7]indazino[1,2-b]quinolin-4-yl(4-((S)-2-(4-(((4methoxyphenyl)diphenylmethyl)amino)butyl)-35-(4-((6-(2-(methylsulfonyl)pyrimidin-5-yl)hexan-5-amido)methyl)-1H-1,2,3-triazol-1-yl)-4,8-dioxo-6,12,15,18,21,24,27,30,33-nonoxy-3,9-diazapentatriacontanamino)benzyl)carbonate

At room temperature, compound 33-1 (14 mg, 0.05 mmol) was dissolved in dimethyl sulfoxide and water (2.0 mL:0.5 mL), cuprous bromide (11 mg, 0.08 mmol) was added, and the mixture was stirred to react for 1 h. The system was purified by preparative HPLC to obtain the title compound (30 mg). ESI-MS (m/z): 815.9[(M-273)/2+H]+.

### Step 4: Synthesis of 4-((S)-2-(4-aminobutyl)-35-(4-((6-(2-(methylsulfonyl)pyrimidin-5-yl)hexan-5-amido)methyl)-1H-1,2,3-triazol-1-yl)-4,8-dioxo-6,12,15,18,21,24,27,30,33-nonaoxa-3,9-diazapentatriacontanamino)benzyl((S)-4-ethyl-11-(2-(N-isopropylmethylsulfonamido)ethyl)-3,14-dioxo-3,4,12,14-tetrahydro-1H-pyrano[3',4':6,7]indolizino[1,2-b]quinolin-4-yl)carbonate (compound IM-1)

Compound 33-2 (30 mg, 0.02 mmol) was dissolved in dichloromethane (1.0 mL) and trifluoroacetic acid (0.2 mL) was added to the reaction to react at room temperature for 30 min. After purification by preparative high performance liquid chromatography (method C), the trifluoroacetate of the title compound was obtained (20.0 mg). Its structure is characterized as follows:
¹H NMR (400 MHz, DMSO-d6) δ 10.18 (s, 1H), 9.10 (s, 2H), 8.38 (t, J = 5.56 Hz, 1H), 8.32 (d, J = 8.40 Hz, 1H), 8.22 - 8.20 (m, 2H), 8.09 (t, J = 5.68 Hz, 1H), 7.91-7.87 (m, 2H), 7.82-7.78 (m, 1H), 7.69 (brs, 3H), 7.61 (d, J = 8.56 Hz, 2H), 7.32 (d, J = 8.56 Hz, 2H), 7.06 (s, 1H), 5.56 (d, J = 16.96 Hz, 1H), 5.51 (d, J = 16.96 Hz, 1H), 5.47 (d, J = 19.28 Hz, 1H), 5.42 (d, J = 19.28 Hz, 1H), 5.14 (d, J = 12.20 Hz, 1H), 5.07 (d, J = 12.16 Hz, 1H), 4.48 (t, J = 5.24 Hz, 2H), 4.46 - 4.43 (m, 1H), 4.29 (d, J = 5.60 Hz, 2H), 4.08 - 3.95 (m, 5H), 3.79 (t, J = 5.28 Hz, 2H), 3.51 - 3.43 (m, 32H), 3.40 (s, 3H), 3.39 - 3.35 (m, 2H), 3.30 - 3.26 (m, 2H), 3.00 (s, 3H), 2.82 - 2.74 (m, 2H), 2.56 (t, J = 7.08 Hz, 2H), 2.29 (t, J = 7.36 Hz, 2H), 2.23 - 2.13 (m, 2H), 1.82 (p, J = 7.24 Hz, 2H), 1.78 - 1.63 (m, 2H), 1.61 - 1.49 (m, 2H), 1.42 - 1.27 (m, 2H), 1.15 (d, J = 6.80 Hz, 3H) , 1.13 (d, J = 6.76 Hz, 3H) , 0.90 (t, J = 7.32 Hz, 3H).
ESI-MS (m/z): 816.0[M/2⁺H]⁺.
[α]_{D}²⁰: -19.55° (c =1.000g/100mL, CH₃CN).

### Example 2. Preparation of conjugate A

0.3 mL of Sacituzumab antibody (anti-Trop-2, 33.5 mg/mL) was taken, diluted with 0.25 mL of a solution (pH 7.6) containing 20 mM PB, 150 mM NaCl and 20 mM sodium edetate, then 0.45 mL of a solution (pH 7.6) containing 20 mM PB and 150 mM NaCl was added and the mixture was mixed well. pH of the reaction mixture was adjusted to 7.4 with 1 M Na₂HPO₄ solution, 10 mM TCEP (tris(2-carboxyethyl)phosphine) solution was added, the mixture was mixed well, and left to stand at room temperature for 30 min. 10 times in moles of IM-1 trifluoroacetate dissolved in dimethyl sulfoxide was added to the above solution system, the system was mixed well, and left to stand at room temperature for 2 h. Thereafter, 6.1 µl of 100 mM cysteine was added to terminate the reaction. Finally, G-25 gel column was used to replace the buffer solution with PBS buffer solution at pH 6.5, and a product of IM-1 coupled with Sacituzumab antibody was obtained, which was named conjugate A.

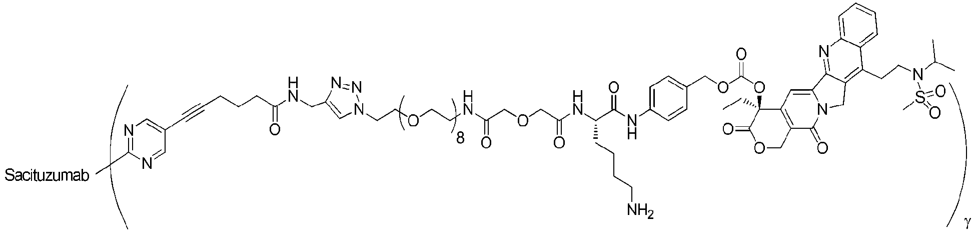

The molecular weight of the conjugate A was analyzed by LCMS, and the measured molecular weight of light chain and heavy chain of the conjugate A was correlated with the theoretical molecular weight of light chain and heavy chain coupled with different numbers of toxins. It was determined that each antibody molecule in the conjugate A was coupled with 1-10 toxins (i.e., γ was 1-10). Then the average coupling ratio (DAR) was calculated to be about 6.9 according to the respective percentages of conjugate molecules coupled with different numbers of toxins.

With reference to Example 2, conjugate A samples with DAR value ranging from 6 to 8 (such as 7.3 or 7.4) were prepared in batches, and the following non-clinical and clinical studies were carried out.

### Example 3. Detection of the inhibitory effect of conjugate on the proliferation of pancreatic cancer cell line

The effect of the conjugate A on the proliferation of BxPC-3 cells (pancreatic cancer cell line, from ATCC, TROP2 positive cells) was detected by the CellTiter-Glo^{®} chemiluminescence cell viability assay (i.e., CTG). On the first day of the experiment, BxPC-3 cells in exponential growth phase were collected, the concentration of the cell suspension was adjusted with the culture medium, and the suspension was added to a 96- well cell culture plate, the final cell concentration being 2000 cells/well. The cells were cultured overnight in a 5% CO₂ incubator at 37°C. On the second day of the experiment, the conjugate A (final concentration 0.152-1000 nM) was diluted to 10-fold dilute working solution with a gradient of 1:3, and 10 µl of the corresponding 10-fold dilute working solution was added to each well, 3 replicate wells for each drug concentration. After adding the drug, the cells were cultured in a 5% CO₂ incubator at 37°C for 72 h. On the fifth day of the experiment, 50 µl (1/2 culture volume) of CTG solution melted in advance and equilibrated to room temperature was added to each well, and the mixture was mixed well with a microplate shaker for 2 min. After standing at room temperature for 20 min, the fluorescence signal value was measured using an Envision 2104 plate reader.

The results show that the conjugate A significantly inhibited the proliferation of TROP2 positive cell BxPC-3, with the IC₅₀ of 14.3 nM, and the maximum inhibition rate of 95.3%. This indicates that the conjugate A can inhibit the proliferation of TROP2-positive pancreatic cancer cells, suggesting that it has therapeutic effects on pancreatic cancer.

### Example 4. Detection of the effects of conjugate on cardiovascular and respiratory functions

The cynomolgus monkeys were divided into 10 (half male and half female)/group. The conjugate A was injected intravenously at doses of 25 mg/kg, 50 mg/kg and 75 mg/kg respectively, once every two weeks, for a total of 4 times. The II-lead ECG and respiratory frequency were detected by a large animal noninvasive physiological signal telemetry system, and arterial blood pressure was measured by a noninvasive sphygmomanometer, so as to evaluate the effects of the conjugate A on cardiovascular and respiratory functions of cynomolgus monkeys.

The results show that prior to the first dosing, 2-3 h, 24-25 h, 72-73 h, 7 d after the first dosing, about 2-3 h after the last dosing and prior to the end of the recovery period, for the monkeys in each group receiving the conjugate A, there was no arrhythmia in II-lead ECG, and heart rate, R-R interval, P-wave time, P-R interval, QRS wave time, Q-T interval, corrected Q-T interval and other parameters of II-lead electrocardiogram, and systolic blood pressure, diastolic blood pressure, mean arterial pressure and respiratory rate were not significantly abnormal. These results indicate that the conjugate A has no effect on the cardiovascular system and respiratory system of cynomolgus monkeys, and has a good prospect of clinical drug safety.

### Example 5. Detection of toxic metabolic behavior of conjugate in animals

The cynomolgus monkeys were divided into four groups according to different doses: control group, 25 mg/kg dose group, 50 mg/kg dose group and 75 mg/kg dose group, with 5 males and 5 females in each group. At the dose levels of 25 mg/kg and 50 mg/kg, the cynomolgus monkeys were injected with the conjugate A intravenously once every two weeks for four consecutive times. For the monkeys in each group, blood samples were collected before and immediately after, and 4 h, 24 h, 48 h, 96 h and 168 h after the first and last dosing (0-1 min after the end of dosing), 1 h before and immediately after the second and third dosing (0-1 min after the end of dosing), and 336 h after the last dosing. For the monkeys in the 75 mg/kg dose group, additionally, blood samples were collected at 4 h, 24 h, 48 h, 96 h and 168 h after the third dosing, so as to detect the toxicokinetic behavior of the conjugate and toxin molecules released by it in vivo.

Table 1 below records the peak plasma concentration (Cₘₐₓ) of and exposure (AUC) to the conjugate A and the toxin molecules released by the conjugate A in male and female monkeys after administration in the above manner. The results show that the highest non-serious toxic dose (HNSTD) of the conjugate A was 50 mg/kg, and after the last dosing with this dose, the exposure to the toxin molecules in female and male monkeys was 3.85 h*µg/mL and 5.86 h*µg/mL respectively, and the exposure to the conjugate A in female and male monkeys was 45.8 h*mg/mL and 64.2 h*mg/mL, respectively.

**Table 1: Peak plasma concentration (Cₘₐₓ) and exposure value (AUC) of Conjugate A and toxin molecule released by Conjugate A in male and female monkeys**

| Dose (mg/kg, QW) | 0 | | 25 | | 50 | | 75 | |
|---|---|---|---|---|---|---|---|---|
| Sex of animal | Male | Female | Male | Female | Male | Female | Male | Female |
| Cₘₐₓ (µg/mL) of conjugate A | | | | | | | | |
| Day 1 | NA | NA | 599 | 641 | 1220 | 1170 | 1820 | 2000 |
| Day 43 | NA | NA | 601 | 518 | 1270 | 1370 | 1790 | 1820 |

| Cₘₐₓ (ng/mL) of toxins | | | | | | | | |
|---|---|---|---|---|---|---|---|---|
| Day 1 | NA | NA | 49.0 | 38.3 | 94.0 | 113 | 132 | 132 |
| Day 43 | NA | NA | 64.5 | 65.1 | 146 | 127 | 110 | 137 |

| AUCₗₐₛₜ (hr*mg/mL) of conjugate A | | | | | | | | |
|---|---|---|---|---|---|---|---|---|
| Day 1 | NA | NA | 25.9 | 26.9 | 63.2 | 46.3 | 87.5 | 85.5 |
| Day 43 | NA | NA | 19.2 | 14.9 | 64.2 | 45.8 | 78.5 | 87.9 |

| AUCₗₐₛₜ (hr*µg/mL) of toxins | | | | | | | | |
|---|---|---|---|---|---|---|---|---|
| Day 1 | NA | NA | 1.79 | 1.43 | 4.49 | 3.97 | 5.42 | 5.12 |
| Day 43 | NA | NA | 2.57 | 1.86 | 5.86 | 3.85 | 4.08 | 4.68 |

According to the experimental results, it can be seen that the exposure or Cₘₐₓ data of ADC and toxins in all of the groups of animals were close after the first and last dosing, indicating that the conjugate A has no obvious toxicity accumulation after continuous intravenous administration, and has good tolerance in animals, so that it has a good prospect for clinical drug safety.

### Example 6. Metabolic stability of conjugate in vitro

In this experiment, firstly, the conjugate A and Trodelvy^{™} were respectively formulated into a 3.4 mg/mL working solution with physiological saline, and then the conjugate A and Trodelvy^{™} working solutions were added into human blank plasma respectively to obtain 0.05 mg/ml human plasma samples. The plasma samples were incubated at 37°C, and the release of toxin molecules was determined at 1 h, 3 h, 24 h, 48 h, 72 h and 144 h. The conjugate A and the commercially available drug Trodelvy^{™} were compared for the difference in plasma stability in vitro. The results are shown in Fig. 1.

As shown in Fig. 1 of the specification, with the increase of incubation time, the yield of free toxins in human plasma increased, and after incubation for 24 h, the release percentage of toxin molecules of the conjugate A and the commercially available ADC drug Trodelvy^{™} in human plasma was 28.5% and 93.4%, respectively; after incubation for 48 h, the release percentage of toxin molecules of the conjugate A and the commercially available ADC drug Trodelvy^{™} in human plasma was 44.6% and 109.1%, respectively; after incubation for 72 h, the release percentage of toxin molecules of the conjugate A and the commercially available ADC drug Trodelvy^{™} in human plasma was 53.7% and 100.6%, respectively; and after incubation for 144 h, the release percentage of toxin molecules of the conjugate A and the commercially available ADC drug Trodelvy^{™} in human plasma was 65.3% and 104.8%, respectively.

The results showed that the release rate of toxin molecules of conjugate A in human plasma was significantly lower than that of Trodelvy^{™}, which indicates that the conjugate A is relatively stable in human plasma, and releases less toxins in plasma, effectively achieving the goal of releasing toxins after reaching the target tumor sites. Moreover, compared with the commercially available drug Trodelvy^{™}, the conjugate A can reduce the risk of serious adverse reactions caused by rapid and excessive release of toxins in plasma.

### Example 7. Clinical trial

### I. Protocol

Patients with malignant tumor of epithelial origin diagnosed by histology were treated with the conjugate A. All the patients had unresectable locally advanced or metastatic solid tumors that had failed standard treatments, or had no standard treatment regimens or were not suitable for standard treatments at the present stage. Based on the patient's weight, a dose escalation design with five dose cohorts of 2, 4, 6, 9 and 12 mg/kg was employed, which were injected intravenously once every two weeks, every 28 days as a cycle, and were given every cycle until the disease progressed or an intolerable toxic reaction occurred. The toxicity of all planned dose groups and some intermediate dose groups was evaluated by BLRM. Based on the model analysis, the sponsor and the researcher jointly decided the decision of dose increase.

### II. Pharmacokinetic study

In the PK analysis of the dose-escalation cohort, the exposure of Conjugate A increased proportionally with the dose within the test dose range of 2 to 6 mg/kg. No accumulation of Conjugate A was observed after multiple administrations. The half-lives of Conjugate A and free payload were approximately 36 hours and 49 hours, respectively, under a biweekly administration schedule. The plasma exposure of free payload (expressed as maximum plasma concentration (Cₘₐₓ) and area under the curve (AUC)) was approximately 6% and 5% of that of Conjugate A in the first 4-week cycle, respectively, while the pharmacokinetic parameters and plasma exposure of total antibodies (referring to conjugated, partially unconjugated, and completely unconjugated antibodies) were similar to those of Conjugate A. These results indicated that the linker of Conjugate A was stable during systemic circulation and that most payload molecules were directed to tumor tissues by the targeting antibodies.

### III. Safety results

During the dose-escalation study, no TEAEs of grade 3 or higher occurred in the 2 mg/kg dose group; TEAEs of grade 3 occurred in the 4 mg/kg dose group, including oral mucositis and anemia; and TEAEs of grade 3 or higher occurred in the 6 mg/kg dose group, including decreased neutrophil count and decreased white blood cell count. 28 (93.9%) patients enrolled in the dose-escalation cohort reported TRAEs, and 17 (56.7%) patients experienced TRAEs of grade 3 or higher, the most common (incidence ≥5%) being anemia (26.7%), neutropenia (23.3%), leukopenia (16.7%), stomatitis (16.7%), and thrombocytopenia (13.3%). The aforementioned TEAEs of grade 3 or higher were recoverable after symptomatic treatment, the administration could be continued, and there were no TRAEs leading to death. Therefore, the results of the dose-escalation study showed that Conjugate A was safe and well tolerated.

In the subsequent dose-expansion study, 211 patients received at least one dose of Conjugate A. Among them, 23 and 188 patients with different types of advanced solid tumors were treated with Conjugate A at dose levels of 4 mg/kg Q2W and 5 mg/kg Q2W, respectively. 202 (95.7%) patients reported TRAEs. 110 (52.1%) patients reported TRAEs of grade 3 or higher, and most of them were recovered after supportive care or dose adjustment. Treatment-related serious adverse events (TRSAEs) occurred in 51 (24.2%) patients, and no TRAEs leading to death were reported. This further suggested that the safety of Conjugate A monotherapy was tolerable and manageable.

The TRAEs at dose levels of 4 mg/kg Q2W and 5 mg/kg Q2W were summarized in Table 2 below.

**Table 2: TRAEs at doses of 4 mg/kg Q2W and 5 mg/kg Q2W**

| Response type | Conjugate A at 4 mg/kg Q2W (N=23) | | Conjugate A at 5 mg/kg Q2W (N=188) | |
|---|---|---|---|---|
| | All grades (n, %) | Grade ≥3 (n, %) | All grades (n, %) | Grade ≥3 (n, %) |
| Any TRAE | 23 (100) | 11 (47.8) | 179 (95.2) | 99 (52.7) |
| Anemia | 17 (73.9) | 4 (17.4) | 136 (72.3) | 44 (23.4) |
| Lymphopenia | 17 (73.9) | 3 (13.0) | 113 (60.1) | 32 (17.0) |
| Neutropenia | 14 (60.9) | 2 (8.7) | 106 (56.4) | 49 (26.1) |
| Naupathia | 10 (43.5) | 0 | 58 (30.9) | 2 (1.1) |
| Emesis | 10 (43.5) | 0 | 50 (26.6) | 1 (0.5) |
| Stomatitis | 6 (26.1) | 1 (4.3) | 82 (43.6) | 16 (8.5) |
| Alopecia | 3 (13.0) | 0 | 60 (31.9) | 0 (0.0) |
| Rash | 6 (26.1) | 0 | 66 (35.1) | 8 (4.3) |
| Thrombocytopenia | 6 (26.1) | 3 (13.0) | 65 (34.6) | 15 (8.0) |

In a phase 1/2 multicenter dose-escalation/expansion study in patients with relapsed or refractory locally advanced/metastatic NSCLC and other tumor types, all NSCLC patients received Conjugate A at a dose of 5 mg/kg IV Q2W. Tumor assessments per RECIST 1.1 were performed every 8 weeks by the researchers. Among the 43 patients enrolled (63% male, 88% ECOG psl, median age 58 years [44-74]), 67.4% (29/43) experienced TRAEs of grade ≥3. The most common TRAEs of grade ≥3 (incidence ≥5%) were decreased neutrophil count (32.6%), anemia (30.2%), decreased white blood cell count (WBC) (23.3%), stomatitis (9.3%), rash (7.0%), and decreased lymphocyte count (7.0%). TRAEs of grade 4 occurred only for neutropenia and leukopenia. Most hematologic toxicities occurred within the first two months of treatment, and were resolved after administration of granulocyte colony-stimulating factor or erythropoietin without blood transfusion. Dose reductions due to TRAEs occurred in 23.3% (10/43) patients. No neuropathy or drug-related ILD/pneumonitis were reported. No TRAEs led to treatment discontinuation or death.

### IV. Efficacy results

The treatment results for various indications are as follows:

### 1. Triple negative breast cancer (TNBC)

### In all the patients with triple negative breast cancer, some patients showed partial response.

A patient who had progressive disease after previous adriamycin/cyclophosphamide/paclitaxel neoadjuvant chemotherapy, left mastectomy and left chest radiotherapy was injected with the conjugate A intravenously at a dose of 4 mg/kg based on the patient's body weight according to the above-mentioned administration cycle. After 18 weeks, partial response was observed, which lasted for 6 weeks. In several times of efficacy evaluation, the total volume of the target lesions could be reduced by 40%. The patient did not have serious adverse events.

### 2. Ovarian cancer

In all the patients with ovarian cancer, some patients showed partial response.

A patient with ovarian cancer who had failed previous hysterectomy, ovarian tumorectomy, carboplatin/paclitaxel, rucaparib, carboplatin/docetaxel/bevacizumab was injected with the conjugate A intravenously at a dose of 4 mg/kg based on the patient's body weight according to the above administration cycle. After 21 weeks, partial response was observed, which lasted for 15 weeks. In several times of efficacy evaluation, the total volume of the target lesions was reduced by 64.8%, and one of the target lesions completely disappeared. The patient did not have serious adverse events.

### 3. HER2 positive breast cancer

A patient with HER2 positive breast cancer who had previously failed modified radical mastectomy, epirubicin/paclitaxel, HER2 monoclonal antibody/docetaxel and capecitabine was injected with the conjugate A intravenously at a dose of 6 mg/kg based on the patient's body weight according to the above administration cycle. After 8 weeks, partial response was observed. In several times of efficacy evaluation, the total volume of the target lesions could be reduced by 49.6%.

### 4. Gastric cancer

A patient with gastric cancer who had previously failed total gastrectomy, paclitaxel/tegafur, anlotinib/tegafur, anlotinib/capecitabine, oxaliplatin/fluorouracil and oxaliplatin/raltitrexed was injected with the conjugate A intravenously at a dose of 4 mg/kg based on the patient's body weight according to the above administration cycle. After 15 weeks, partial response was observed, which had lasted for 10 weeks and was still benefiting. In several times of efficacy evaluation, the total volume of the target lesions could be reduced by 62.8%, and one of the target lesions disappeared.

No serious adverse events occurred in the patient.

### 5. Pancreatic cancer

A patient with pancreatic cancer who had previously failed multi-line therapy of distal resection of pancreatic cancer, radiotherapy, gemcitabine/capecitabine, gemcitabine/irinotecan/fluorouracil, gemcitabine/albumin paclitaxel/oxaliplatin/fluorouracil, and nivolumab/cabiralizumab was injected with the conjugate A intravenously at a dose of 4 mg/kg based on the patient's body weight according to the above-mentioned administration cycle, and stable disease was observed after 7 weeks, which lasted for 30.3 weeks. In several times of efficacy evaluation, the total volume of the target lesions could be reduced by 8.8%, and no serious adverse events occurred in the patient.

### 6. HR+/Her2- breast cancer

39 patients were enrolled and treated with Conjugate A. Among the 28 patients who were evaluable for response (patients with at least one post-baseline tumor assessment), the ORR was 42.9% (12/28, 2 cases to be confirmed) and the DCR was 85.7% (24/28), with a median PFS of 7.6 months.

### 7. Non-small cell lung cancer (NSCLC)

In a phase 1/2 multicenter dose-escalation/expansion study for patients with relapsed or refractory locally advanced/metastatic NSCLC and other tumor types, all NSCLC patients received Conjugate A at a dose of 5 mg/kg IV Q2W. The tumor assessments per RECIST 1.1 were performed by researchers every 8 weeks.

Among the 43 patients enrolled (63% male, 88% ECOG ps1, median age 58 years [44-74]), the median follow-up time was 11.5 months (mo; 95% ci, 10.4 to 12.2). The median treatment duration was 5.7 months (range 0.5 to 14.1). Among the 39 patients who were evaluable for response, the ORR was 44% (17/39, 15 confirmed and 2 pending), the median DoR was 9.3 months (range, 1.3+ to 11.2+), and the 6-month DoR rate was 77%. For the EGFR wild-type subgroup (previously received a median of two lines of therapy, including anti-PD-1/L₁ monoclonal antibody therapy), the ORR was 26% (5/19), the DCR was 89% (17/19), the median PFS was 5.3 months, and the 9-month OS rate was 80.4%. For the TKI-resistant EGFR mutant NSCLC subgroup (50% also failed at least one line of chemotherapy), the ORR was 60% (12/20), the DCR was 100% (20/20), the median PFS was 11.1 months, and the 9-month PFS rate was 66.7%.

In summary, the clinical trials showed that Conjugate A had a good efficacy in the above-mentioned unresectable locally advanced or metastatic solid tumors that have failed standard treatment, and did not induce severe toxicity that could hinder clinical use. These results proved that Conjugate A had good safety and efficacy for the treatment of tumor diseases, and that Conjugate A prepared in different batches with a DAR value range of 6 to 8 (e.g., DAR value of 7.3 or DAR value of 7.4) had substantially consistent efficacy and safety profiles.

Various modifications of the present invention other than those described herein will be apparent to those skilled in the art from the foregoing description. Such modifications are also intended to fall within the scope of the appended claims. All the reference documents cited in the present application (including all the patents, patent applications, journal articles, books and any other publications) are incorporated herein by reference in their entirety.

## Claims

1. Use of a bioactive molecule conjugate represented by Formula (I) in the manufacture of a medicament for treating a subject with a recurrent or refractory locally advanced or metastatic non-small cell lung cancer;
{D-[L₁-(L₂)ₘ₁-(L₃)ₘ₂-(L₄)ₘ₃-E]}_{γ}-^{A} Formula (I)
wherein,
L₁ is 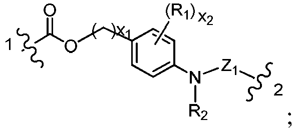 wherein, each R₁ and R₂ is independently hydrogen (e.g., protium or deuterium), halogen, carboxylic acid group, sulfonic acid group, cyano, C₁₋₆ alkyl, halogenated C₁₋₆ alkyl, cyano-substituted C₁₋₆ alkyl (e.g., -CH₂CN), C₁₋₆ alkoxy, C₂₋₁₀ alkenyl or C₂₋₁₀ alkynyl; Z₁ is an amino acid or a peptide composed of 2 to 10 amino acids; x₁ and x₂ are each independently 0, 1, 2, 3, 4, 5 or 6; and L₁ is connected to D at position 1 and connected to L₂ at position 2;
L₂ is 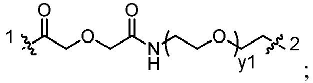 wherein y₁ is 0, 1, 2, 3, 4, 5, 6, 7, 8, 9 or 10; and L₂ is connected to L₁ at position 1 and connected to L₃ at position 2;
L₃ is selected from 5- to 12-membered heteroaromatic ring;
L4 is 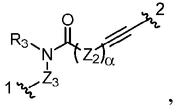 wherein Z₂ is selected from the group consisting of C₁₋₆ alkylene, C₂₋₁₀ alkenylene, C₂₋₁₀ alkynylene, and C₃₋₈ cycloalkylene; R₃ is selected from the group consisting of H and C₁₋₆ alkylene; Z₃ is absent or selected from C₁₋₆ alkylene; or, R₃ and Z₃ together with the nitrogen atom to which they are attached form a 4- to 8-membered heterocyclyl; α is 0, 1, 2, 3, 4, 5 or 6, and the position 2 of L₄ is connected to E, and the position 1 of L₄ is connected to L₃;
E is 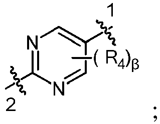 wherein each R₄ is independently hydrogen (e.g., protium or deuterium), β is 0, 1 or 2, and the position 2 of E is connected to A (e.g., connected to the thiol group on A), and the position 1 of E is connected to L₄;
m₁, m₂ and m₃ are each independently 0, 1, 2, 3, 4, 5, 6, 7, 8, 9 or 10;
D is a biologically active molecule fragment;
γ is selected from an integer between 1 and 10; preferably, γ is selected from an integer between 3 and 8 (e.g., 3, 4, 5, 6, 7 or 8);
A is an anti-Trop-2 monoclonal antibody or an antigen-binding fragment thereof.

2. The use according to claim 1, wherein the non-small cell lung cancer is an unresectable locally advanced or metastatic non-small cell lung cancer that has failed standard treatment, or has no standard treatment option, or is currently unsuitable for standard treatment.

3. The use according to claim 1 or 2, wherein the non-small cell lung cancer is EGFR wild-type non-small cell lung cancer.

4. The use according to claim 3, wherein the subject has previously received a median of two lines of therapy.

5. The use according to claim 3, wherein the subject has previously received platinum-based chemotherapy combined with anti-PD-1/L1 monoclonal antibody therapy.

6. The use according to claim 1 or 2, wherein the non-small cell lung cancer carries an EGFR mutation, preferably, the EGFR mutation includes exon 19 deletions or exon 21 L858R mutations.

7. The use according to claim 6, wherein the subject with EGFR-mutated NSCLC has previously received treatment with a first-generation or second-generation epidermal growth factor receptor-tyrosine kinase inhibitor (EGFR-TKI), and confirmed by tumor biopsy that exon 20 T790M mutation is negative after treatment failure, or the subject with EGFR-mutated NSCLC has previously received treatment with a third-generation EGFR-TKI, irrespective of the presence of T790M mutation.

8. The use according to claim 6 or 7, wherein the non-small cell lung cancer is resistant to EGFR-TKI treatment.

9. The use according to any one of claims 6 to 8, wherein the subject has received first-line treatment, preferably, the first-line treatment is platinum-based chemotherapy.

10. The use according to any one of claims 1 to 9, wherein the conjugate has the following structure:
L₁ is selected from the group consisting of 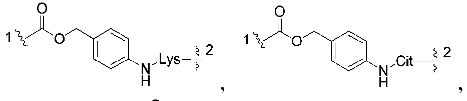 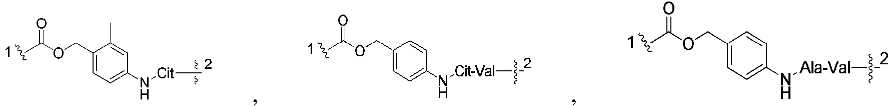 and 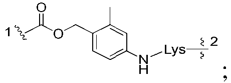 and the position 1 of L₁ is connected to D and the position 2 of L₁ is connected to L₂;
L₂ is 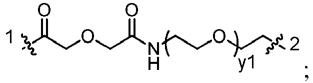 wherein y1 is 3, 4, 5, 6, 7, 8, 9 or 10; and the position 1 of L₂ is connected to L₁, and the position 2 of L₂ is connected to L₃;
L₃ is selected from a 5- to 6-membered heteroaromatic ring, such as pyrazole or triazole;
L₄ is 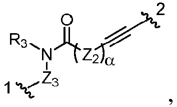 wherein Z₂ is selected from C₁₋₃ alkylene; R₃ is H; Z₃ is selected from C₁₋₃ alkylene; α is 1, the position 2 of L₄ is connected to E, and the position 1 of L₄ is connected to L₃;
E is 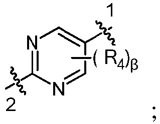 wherein each R₄ is independently hydrogen (e.g., protium or deuterium), β is 0, 1 or 2, the position 2 of E is connected to A (e.g., connected to the thiol group on A), and the position 1 of E is connected to L₄;
m₁, m₂ and m₃ are all 1;
the bioactive molecule is selected from the group consisting of 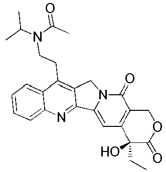 and 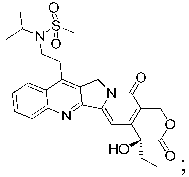 preferably, the bioactive molecule is connected through its own hydroxyl group to the position 1 of L₁;
γ is selected from an integer between 3 and 8 (e.g., 3, 4, 5, 6, 7 or 8);
A is Sacituzumab or an antigen-binding fragment thereof.

11. The use according to any one of claims 1 to 10, the conjugate has the following structure: 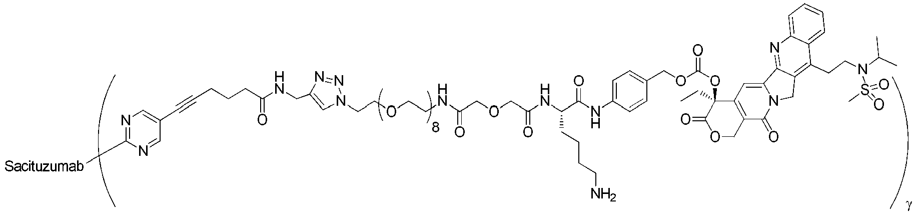 wherein, γ is an integer between 1 and 10; preferably, γ is selected from an integer between 5 and 8.

12. A method for treating recurrent or refractory locally advanced or metastatic non-small cell lung cancer, the method comprising a step of administering to a subject in need thereof a therapeutically effective amount of a bioactive molecule conjugate as described in any one of claims 1, 10 and 11 and/or a pharmaceutical composition comprising a bioactive molecule conjugate as described in any one of claims 1, 10 to 11.

13. The method according to claim 12, wherein the non-small cell lung cancer is an unresectable locally advanced or metastatic non-small cell lung cancer that has failed standard treatment, or has no standard treatment regimen option, or is currently unsuitable for standard treatment.

14. The method according to claim 12 or 13, wherein the non-small cell lung cancer is EGFR wild-type non-small cell lung cancer.

15. The use according to claim 14, wherein the subject has previously received a median of two lines of therapy.

16. The method according to claim 14, wherein the subject has previously received platinum-based chemotherapy combined with anti-PD-1/L₁ monoclonal antibody therapy.

17. The method according to claim 12 or 13, wherein the non-small cell lung cancer carries an EGFR mutation, preferably, the EGFR mutation includes exon 19 deletions or exon 21 L858R mutations.

18. The method according to claim 17, wherein the subject with EGFR-mutated NSCLC has previously received treatment with a first-generation or second-generation epidermal growth factor receptor-tyrosine kinase inhibitor (EGFR-TKI), and confirmed by tumor biopsy that the exon 20 T790M mutation is negative after treatment failure, or the subject with EGFR-mutated NSCLC has previously received treatment with a third-generation EGFR-TKI, irrespective of the presence of T790M mutation.

19. The method according to claim 17 or 18, wherein the non-small cell lung cancer is resistant to EGFR-TKI treatment.

20. The method according to claim 19, wherein the subject has received first-line treatment, preferably, the first-line treatment is platinum-based chemotherapy.

21. The method according to any one of claims 12 to 20, wherein the pharmaceutical composition comprises the bioactive molecule conjugate and a pharmaceutically acceptable carrier and/or excipient.

22. The method according to any one of claims 12 to 21, wherein the bioactive molecule conjugate or the pharmaceutical composition is administered once every 7 to 35 days, preferably once every 7 to 28 days, for example, once every 7 days, 14 days, 21 days, 28 days, or 35 days.

23. The method according to any one of claims 12 to 22, wherein the bioactive molecule conjugate or the pharmaceutical composition is administered once every 14 days.

24. The method according to any one of claims 12 to 23, wherein the conjugate or the pharmaceutical composition is administered by the following routes: oral administration, transdermal injection, rectal administration, transmucosal administration, intramuscular injection, intramedullary injection, intravenous injection, intraperitoneal injection, preferably intravenous injection.

25. The method according to any one of claims 12 to 24, wherein the dose of the bioactive molecule conjugate for each administration based on the subject's body weight is 1 mg/kg to 30 mg/kg; preferably 1 mg/kg to 20 mg/kg; more preferably 2 mg/kg to 12 mg/kg; further preferably 2 to 5 mg/kg, 4 to 7 mg/kg, 6 to 9 mg/kg, 8 to 11 mg/kg, 10 to 13 mg/kg, or 12 to 15 mg/kg.

26. The method according to any one of claims 12 to 25, wherein the dose of the bioactive molecule conjugate for each administration based on the subject's body weight is 4 mg/kg or 5 mg/kg.

27. The method according to any one of claims 12 to 26, wherein the dose of the bioactive molecule conjugate or its composition for each administration based on the subject's body weight is 1 mg/kg to 20 mg/kg, and the administration is carried out once every 7 to 28 days.

28. The method according to any one of claims 12 to 27, which is divided into one or more administration stages (e.g., one, two, three or four stages), wherein the administration cycle of each stage is as described in any one of claims 22, 23, 27, and/or the dose for administration of each stage is as described in any one of claims 25 to 27.
